# EUROPÄISCHE PATENTANMELDUNG

(11) **EP 3 300 749 A1**
(43) Veröffentlichungstag der Anmeldung: **04.04.2018**
(21) Anmeldenummer: 16191579.8
(22) Anmeldetag: 29.09.2016
(51) Int. Cl.: A61M 1/10

(54) **PASSIV MAGNETISCH GELAGERTE BLUTPUMPE**

(71) Anmelder: Berlin Heart GmbH, 12247 Berlin (DE)
(72) Erfinder: GRANEGGER, Marcus, CH-8057 Zürich (CH); WINTERWERBER, Kim Peter, 12357 Berlin (DE); RICHERT, Hendryk, 12487 Berlin (DE); SCHMIDT, Bodo, 14513 Teltow (DE)
(74) Vertreter: Pfenning, Meinig & Partner mbB

(57) **Zusammenfassung**

Das vorliegende Schutzrecht betrifft unter anderem eine Blutpumpe (1), enthaltend einen Einlass (10), einen Auslass (11) sowie einen Rotor (2) zur Förderung von Fluid vom Einlass (10) zum Auslass (11), wobei der Rotor (2) innerhalb der Blutpumpe (1) radial passiv magnetisch gelagert ist und axial in eine Richtung (11) zumindest passiv magnetisch vorgespannt ist, so dass bei fluidfördernder Drehung des Rotors (2) der Axialschub des Rotors entgegen der axial in Richtung des Auslasses wirkenden magnetischen Anziehung wirkt.

## Beschreibung

Eine wichtige Eigenschaft moderner Blutpumpen ist der Grad der Blutschädigung. Je nach Pumpenkonzept und insbesondere Lagerkonzept kann es hier zu unterschiedlich großen Blutschädigungen kommen. Angestrebt ist einerseits eine möglichst geringe Blutschädigung, etwa durch möglichst strömungsgünstige Formen, große Spaltmaße etc. Andererseits sollte die Größe der Pumpe, insbesondere bei voll implantierbaren Pumpen, begrenzt sein. Außerdem ist es erstrebenswert, die Größe der Steuerungseinheit, welche für die Drehzahlregelung sowie die Lagerkontrolle ausgebildet ist, möglichst klein zu halten und auch den Stromverbrauch hierfür gering zu halten.

Momentan gibt es bei klinisch verwendeten Rotationsblutpumpen verschiedene Lagertypen:
1. Mechanische Lager (kommerzielle Beispiele: Heartmate II, Reliant Heart)
2. Hydrodynamische Lager (kommerzielles Produkt: Ventrassist)
3. Hybride Lager (Hydrodynamisch und passiv magnetisch, kommerzielle Beispiele: HVAD, MVAD)
4. Aktive (in ein- oder mehreren Richtungen) Magnetlager (kommerzielle Beispiele: INCOR, HM III).

Alle momentanen Lagerkonfigurationen haben Vor- und Nachteile. Obwohl z.B. das aktive Magnetlager theoretisch ideal in Bezug auf die Blutschädigung ist, ist die Realisierung oftmals komplex (beispielsweise in Bezug auf die Steuereinheit und den Stromverbrauch) und mit zusätzlichem Wärmeeintrag bzw. auch mit substantiellen Sekundärströmungen verknüpft. Andererseits sind hydrodynamische/hybride Lager komplett passiv; dies ermöglicht den Einbau einer kleinen Steuereinheit und einen geringen Stromverbrauch. Die Hämokompatibilität (bzw. Blutschädigung) dieser Systeme ist allerdings aufgrund der großflächig ausgeführten sehr kleinen Spaltmaße technisch nicht unbedingt vorteilhaft und vor allem bei Axialpumpen auch schwer umsetzbar.

Ausgehend von diesem Stand der Technik ist es die Aufgabe des vorliegenden Schutzrechts, eine Blutpumpe bereitzustellen, die einerseits kleinbauend und stromsparend ist und andererseits eine sehr geringe Blutschädigung mit sich bringt.

Diese Aufgabe wird durch eine Blutpumpe nach den Patentansprüchen gelöst.

Dies ist zunächst einmal eine Blutpumpe, enthaltend einen Einlass, einen Auslass sowie einen Rotor zur Förderung von Fluid, insbesondere Blut, vom Einlass zum Auslass, wobei der Rotor innerhalb der Blutpumpe radial passiv magnetisch gelagert ist und axial in eine Richtung zumindest passiv abgestoßen oder angezogen wird. Die Blutpumpe ist beispielsweise so konfiguriert, dass bei fluidfördernder Drehung des Rotors der Axialschub des Rotors entgegen der axial in Richtung des Auslasses wirkenden magnetischen Anziehung wirkt.

Auf diese Weise ist ein passives System bereitgestellt, das in Hinblick auf die Steuerung und den entsprechenden Stromverbrauch sowie die geringe Größe der entsprechenden Komponenten sehr vorteilhaft ist. Durch den bei Rotoren mit Schaufeln bzw. Wendeln oder auch anderen Förderelementen entstehenden Axialschub wird der Rotor in Richtung des Einlasses gezogen. Diese Kraft wirkt der beispielsweise rein magnetisch axial passiv wirkenden Magnetkraft entgegen. Auf diese Weise kommt es zu einer Entlastung eines mechanischen Lagers, so dass der Rotor möglichst kräftefrei, idealerweise sogar "schwebend" gelagert ist. Alternativ kann die magnetische Vorspannung so stark gewählt sein, dass in keinem vorstellbaren Szenario der mechanische Kontakt zwischen Rotor und Stator verlorengeht.

Der Antrieb des Rotors erfolgt beispielsweise berührungslos durch einen bürstenlosen Gleichstrommotor.

Eine Weiterbildung sieht vor, dass der berührungslose Anteil der Lagerung des Rotors rein passiv magnetisch erfolgt, d.h., dass keine zusätzlichen Steuerspulen etc. zur Positionierung des Rotors notwendig sind. Alternativ ist es jedoch möglich, dass beispielsweise zur Verstärkung des Axialschubs des Rotors (also beispielsweise entgegen der Förderrichtung des Fluids) eine auf den Rotor wirkende elektromagnetische und/oder elektrodynamische Vorrichtung vorgesehen ist. Dies ist beispielsweise zur weiteren Entlastung des mechanischen Lagers möglich, um den Rotor möglichst schwebend bzw. kraftarm zu halten. Dies kann beispielsweise durch entsprechende elektromagnetische Vorrichtungen bzw. Steuerspulen geschehen.

Eine Weiterbildung sieht vor, dass der Rotor, insbesondere zur axialen Lagerung, zusätzlich durch ein mechanisches Lager gelagert ist. Dieses mechanische Lager kann insbesondere als Kontaktlager, insbesondere als Spitzen- oder Balllager, ausgeführt sein. Hierdurch wird eine mechanisch verlässliche Vorrichtung geschaffen, die insbesondere auch bei niedrigeren Drehzahlen eine stabile Rotorlage sicherstellt.

Alternativ oder auch zusätzlich kann der Rotor, insbesondere zur axialen Lagerung, zusätzlich durch ein hydrodynamisches Lager unterstützt sein, das beispielsweise auch im auslassseitigen Bereich des Rotors angeordnet ist. Vorteilhafte Beispiele sind hydrodynamische Lager in Form von Spiralrillenlagern.

Eine Weiterbildung sieht vor, dass ein zusätzliches Lager, insbesondere ein axiales Fanglager zur Begrenzung der axialen Rotorbewegung, vorgesehen ist. Hierdurch wird sichergestellt, dass bei einem "Ansaugen" der Pumpe an einer Herzwand und dem damit einhergehenden sehr starken Axialschub keine Beschädigung der Rotorblätter bzw. Blutkontaktflächen der Pumpe dadurch entsteht, dass der Rotor zu stark in Richtung des Einlasses gezogen wird.

Eine Weiterbildung sieht vor, dass im Bereich mindestens eines zusätzlichen Lagers der Rotor und/oder angrenzende Teile der Blutpumpe, insbesondere mit keramischen Materialien und/oder einer Hartstoffbeschichtung, beispielsweise DLC (Diamond-Like Carbon), verstärkt sind.

Blutpumpen gemäß den obigen Ansprüchen sind für verschiedene Lagerungskonzepte möglich, beispielsweise für Axialpumpen/Halbaxialpumpen (mixed flow pumps) oder auch Radialpumpen. Beispielsweise kann eine axiale Einströmung und Förderung durch den Rotor erfolgen, und eine axiale Ausströmung oder auch tangentiale Ausströmung im Bereich des Auslasses. Allen Konzepten gemeinsam ist, dass der Einlass und der Auslass jeweils mit menschlichen oder tierischen Blutgefäßen verbindbar sind, um so den natürlichen Blutfluss zu verstärken und/oder zu regeln.

Im Folgenden werden beispielhaft weitere Aspekte des vorliegenden Schutzrechts erläutert:
Eine Ausführungsform einer erfindungsgemäßen Blutpumpe sieht vor, dass die Blutpumpe eine voll in einen menschlichen oder tierischen Körper implantierbare Pumpe ist. Vorzugsweise ist dies eine Pumpe, die zur Verstärkung und/oder Regelung des Blutkreislaufs des Körpers geeignet ist. Vorzugsweise ist hierbei der Einlass der Pumpe axial bezüglich einer Drehachse des Rotors und der Auslass bezüglich der Drehachse des Rotors radial, und/oder der Rotor der Blutpumpe ist axial, halbaxial oder radial ausgestaltet. Auf diese Weise können auch unterschiedliche Arten von Rotoren auf einfache und sichere Weise gelagert und stabilisiert werden.

Eine weitere Ausführungsform sieht vor, dass Lagermagneten des Rotors zur radialen und axialen Stabilisierung des Rotors innerhalb des Gehäuses der Blutpumpe vorgesehen sind. Das heißt, dass sich mit dem Rotor mitdrehende Lagermagnete vorgesehen sind, die die stabilisierende und/oder zentrierende Funktion für den Rotor übernehmen.

Eine weitere Ausführungsform sieht vor, dass die Lagermagnete des Rotors auf der dem Einlass abgewandten Seite des Rotors angeordnet sind und/oder dass die Lagermagnete auf der dem Einlass zugewandten Seite angeordnet sind. Je nachdem, ob der Rotor axial, halbaxial oder auch radial ausgelegt ist, bzw. abhängig von den Strömungsverhältnissen wird eine größtmögliche Flexibilität bei der Konstruktion der Blutpumpe ermöglicht.

Eine weitere Ausführungsform sieht vor, dass Motorspulen im Stator der Blutpumpe und Rotormagnete im Rotor vorgesehen sind, wobei die Motorspulen auf der dem Einlass zugewandten Seite des Rotors und/oder auf der dem Einlass abgewandten Seite des Rotors angeordnet sind. Auch auf diese Weise ist die größtmögliche Flexibilität bei der Auslegung der Blutpumpe gewährleistet.

Eine weitere Ausführungsform sieht vor, dass ein mechanisches Lager auf der dem Einlass zugewandten Seite des Rotors und/oder auf der dem Einlass abgewandten Seite des Rotors angeordnet ist. Auf diese Weise kann der Rotor auf unterschiedliche Weise gelagert sein (vorzugsweise entgegen der Strömungsrichtung des durch den Einlass einfließenden Bluts), es können jedoch auch andere Anordnungen vorgesehen sein, beispielsweise eine Anordnung mit zwei Lagern, bei der ein Verschluss des Einlasses durch einen in Richtung des Einlasses sich bewegenden Rotor verhindert wird.

Eine weitere Weiterbildung sieht vor, dass der Rotor in seinem zentralen Bereich über Streben mit einem mechanischen Lager verbunden ist. Dies bietet sich beispielsweise für Ausgestaltungen an, in denen nur ein Lager vorgesehen ist und dieses Lager auf der dem Einlass zugewandten Seite des Rotors vorgesehen ist (aber auch auslassseitig kann der Lagerpunkt mit Streben mit dem Rotor verbunden sein).

Eine weitere Ausführungsform sieht vor, dass die Blutpumpe einen Stator aufweist, wobei diese Ausführungsform im Bereich einer Drehachse des Rotors Lagermagnete (diese stehen vorzugsweise mit korrespondierenden Lagermagneten des sich drehenden Rotors in Verbindung) und wobei der Stator im Bereich der Drehachse des Rotors außerdem ein Statorelement aufweist, auf dessen Spitze eine Kalotte eines mechanischen Lager angeordnet ist, und zusätzlich Rotormagnete des Rotors (vorzugsweise in einer Tragscheibe des Rotors) angeordnet sind und/oder mit den Rotormagneten in Wirkverbindung stehende Motorspulen im Stator auf der dem Einlass abgewandten Seite des Rotors angeordnet sind.

Eine weitere Ausführungsform sieht vor, dass die Blutpumpe als Radialpumpe mit Streben ausgeführt ist, wobei auf einer Erhebung des Stators im Bereich der Drehachse des Rotors eine mit Streben des Rotors in Verbindung stehende Kalotte eines mechanischen Lagers angeordnet ist und Lagermagnete einerseits statorseitig in dem Stator angeordnet sind und andererseits im Rotor angeordnete Lagermagnete, vorzugsweise auf der dem Einlass abgewandten Seite des Rotors, angeordnet sind und weiterhin der Rotor zusätzlich Rotormagnete auf der dem Einlass zugewandten Seite des Rotors aufweist, wobei diese Rotormagnete mit Motorspulen im Bereich des Einlasses der Blutpumpe zusammenwirken.

Eine weitere Ausführungsform sieht vor, dass der Rotor passiv magnetisch angezogen ist. Alternativ können (siehe Figuren) auch noch andere Konfigurationen vorgesehen werden.

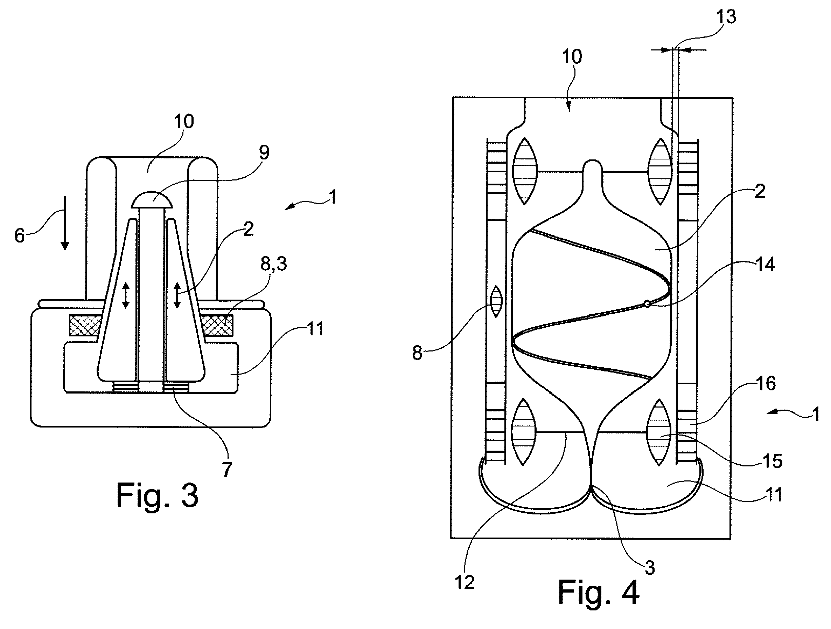

Eine weitere Ausführungsform sieht vor, dass der Radialabstand zwischen mindestens einem rotorseitigen Lagermagneten und mindestens einem statorseitigen Lagermagneten derart klein ist, dass eine sichere Lagerung auch mit möglichst geringem Volumen magnetisch wirksamen Materials erreicht wird. Der fluiddurchlässige Spalt kann hierbei ≥ 50 µm betragen, z.B. 100 µm (siehe z.B. auch Fig. 4, Bezugszeichen 13 in vorliegendem Schutzrecht). Der Spalt zwischen den magnetisch wirksamen Teilen kann z.B. zwischen 500 µm und 2000 µm betragen, z.B. 1000 µm, wobei dies kein Luftspalt sein muss; nichtmagnetische Materialien (z.B. entsprechende Kunststoffe) können in diesem Spalt vorhanden sein; zu Details bzw. Definitionen wird auf die Beschreibungseinleitung dieses Schutzrechts verwiesen).

Eine weitere Ausführungsform sieht vor, dass die Blutpumpe kein dem Rotor vorgelagertes Vorleitrad aufweist. Bei Ausführungsformen, bei denen lediglich ein Lager vorgesehen ist, das den Rotor entgegen der Strömungsrichtung stützt, kann eine solche (baulich einfache) Ausführungsform gewählt werden, insbesondere dann, wenn durch die magnetische Anordnung der Lagermagnete bzw. durch eine geeignete Interaktion der Motorspulen mit den zum Antrieb dienenden Rotormagneten des Rotors innerhalb der Blutpumpe gesichert ist.

Eine weitere Ausführungsform sieht vor, dass die Lagermagnete des Rotors in mit Streben am Rotorgrundkörper befestigten Auslegern des Rotors befestigt sind, wobei diese rotorseitigen Lagermagnete mit radial außerhalb der Lagermagnete des Rotors liegenden Lagermagneten des Stators zur radialen und/oder axialen Stabilisierung des Rotors innerhalb der Blutpumpe in Wechselwirkung stehen.

Eine weitere Weiterbildung sieht vor, dass der Motor zum Antrieb des Rotors als Scheibenmotor ausgeführt ist.

Ein weiterer Aspekt betrifft die Platzlerung von Permanentmagneten in den Schaufeln des Rotors. Hierdurch ergibt sich eine Verringerung des magnetischen Luftspalts. Für sämtliche in dieser Schutzrechtsanmeldung gezeigten Ausführungsformen sollen daher Schaufeln aus magnetischem Material und/oder Schaufeln mit darin enthaltenen Permanentmagneten hiermit als offenbart gelten (egal, welche Lagerungsart vorgesehen ist).

Die Erfindung wird nun anhand mehrerer Figuren erläutert. Es zeigen:
- Fig. 1: einen Querschnitt eines Lagers mit einer Magnetkonfiguration, die eine radiale passive Magnetkraft und eine Vorspannung in axialer Richtung ermöglicht,
- Fig. 2: ein Beispiel einer Blutpumpe, die im auslassseitigen Bereich des Rotors eine zusätzliche mechanische Lagerung aufweist,
- Fig. 3: ein weiteres Beispiel einer Blutpumpe, die im auslassseitigen Bereich des Rotors ein hydrodynamisches Lager aufweist,
- Fig. 4: ein weiteres Beispiel einer Blutpumpe, die im auslassseitigen Bereich des Rotors ein mechanisches Lager aufweist. Diese Pumpe hat die Magnete derart positioniert, dass die Pumpe ohne Vorleitrad auskommt. Um möglichst hohe Steifigkeiten zu erreichen, sind die rotorseitigen Ringmagnete möglichst nahe am Pumpenrohr mit den interagierenden Magneten angebracht. Die Ringmagnete sind durch Streben mit dem Rotor verbunden bzw. an den Schaufeln angebracht.

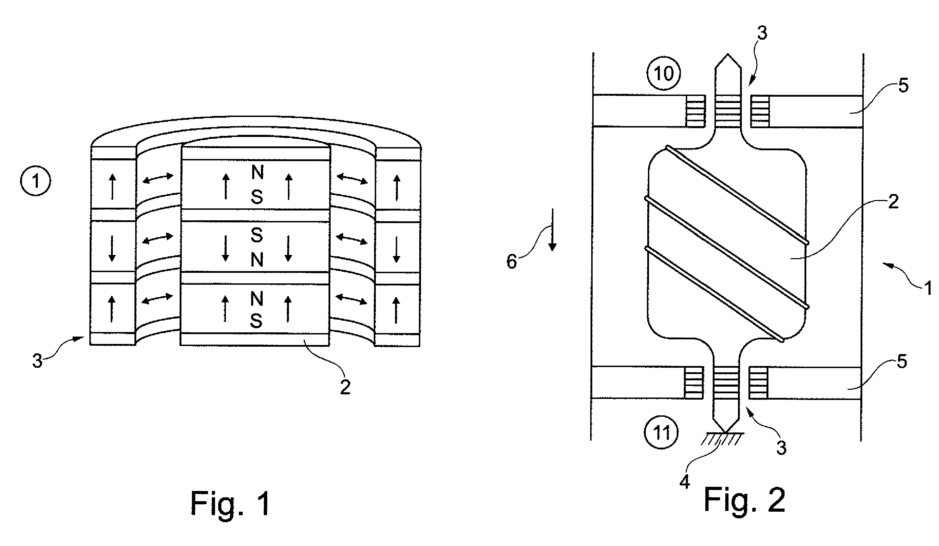

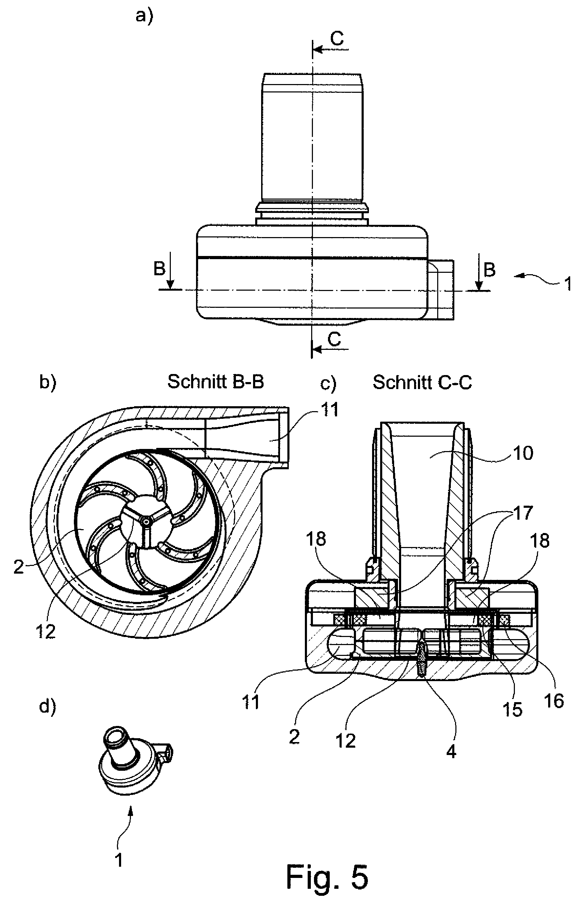

- Fign. 5 bis 7: Beispiele für radiale Blutpumpen, die ein mechanisches Lager aufweisen und magnetisch entgegengesetzt zum Einlass vorgespannt sind. Auch in diesen Ausführungen kann die Ringmagnetkonfiguration gleich der in Fig. 1 beschriebenen sein. Auch hier wird in Ausführungsbeispielen durch den Axialschub Kraft vom mechanischen Lager weggenommen. Das mechanische Lager ist z.B. durch dünne, sich mit dem Rotor drehende Streben mit einer Spindel in der Mitte der Pumpe verbunden. In Beispielen ist der Motor als Scheibenmotor ausgeführt, und die Motormagnete sind in der unteren Deckscheibe untergebracht. Die Pumpe kann auch mit einer oberen Deckscheibe ausgeführt werden, um zusätzlich, abstoßende mit dem Gehäuse interagierende Magnetkräfte auf den Rotor ausüben zu können. Eine derartige Pumpe sollte immer einen mechanischen Kontakt des Kontaktlagers gewährleisten.

Fig. 1 zeigt eine Blutpumpe 1 im Bereich eines Lagers 3. Die in Fig. 1 gezeigten Pfeile deuten die Orientierung der geschichteten Magneten an; dies ist jedoch rein beispielhaft und ohne Festlegung auf eine resultierende Magnetkraftrichtung zu verstehen. Insbesondere ist in Fig. 1 zu sehen, wie ein Teil eines Rotors 2 in einem Ring des Lagers 3 durch eine radiale passive Magnetkraft radial gehalten und eine Vorspannung in eine gewünschte axiale Richtung erreichbar ist. Beispiele für die Anwendung einer solchen Lagerung werden in den folgenden Figuren gezeigt.

Fig. 2 zeigt eine solche Blutpumpe 1, in der ein Rotor 2 gelagert ist. Hierzu sind im Bereich des Einlasses 10 und des Auslasses 11 jeweils an Streben (Vorleitrad bzw. Diffusor) 5 gehaltene Lager 3 (z.B. gemäß Fig. 1) vorgesehen. Durch die Drehung des Rotors entsteht ein Fluss (d.h. eine Fluidströmung) in Richtung des Pfeils 6. Der Rotor wird angetrieben durch einen hier nicht dargestellten Motor.

Die Blutpumpe 1 enthält also einen Einlass 10 sowie einen Auslass 11, wobei der Rotor 2 zur Förderung von Fluid (in Richtung 6) vom Einlass zum Auslass ausgebildet ist. Der Rotor 2 ist innerhalb der Blutpumpe 1 radial passiv magnetisch gelagert und axial in Richtung des Auslasses 11 zumindest passiv magnetisch angezogen (vorgespannt), so dass bei fluidfördernder Drehung des Rotors 2 der Axialschub des Rotors entgegen der wirkenden magnetischen Vorspannung wirkt. Das heißt, dass der Fluss vom Einlass 10 zum Auslass 11 in Richtung 6 erfolgt; der Axialschub des Rotors wirkt in entgegengesetzter Richtung.

In Fig. 2 ist zusätzlich noch eine mechanische Lagerung im auslassseitigen Bereich des Rotors gezeigt, die als Kontaktlager 4 (hier als Spitzenlagerung ausgeführt) vorhanden ist. Hier wird, beispielsweise bei geringen Drehzahlen, eine exakte Rotorpositionierung bzw. -zentrierung bei vergleichsweise niedriger Reibung ermöglicht. Im Bereich des Spitzenlagers ist ein Teil des Rotors bzw. angrenzender Flächen der Blutpumpe aus harten Materialien gefertigt (z.B. Juwelen bzw. Rubin, Saphir, Diamant, DLC oder Keramiken) um hier die Reibung weiter zu minimieren und die Haltbarkeit zu erhöhen.

Fig. 3 zeigt eine alternative Bauform einer Blutpumpe. Hier ist wiederum ein axialer Einlass 10 gezeigt. Außerdem ist eine Auslaufkammer bzw. ein Auslass 11 gezeigt, die eine tangentiale/seitliche Ausströmung von Blut ermöglicht. Der Rotor 2 ist in einem Lager 3 (siehe Fig. 1) magnetisch radial gelagert und axial in eine Richtung vorgespannt. Der Antrieb des Rotors erfolgt berührungsfrei durch einen Motor 8. Gezeigt ist außerdem ein Fanglager 9, das bei einem Ansaugen des Einlasses 10 an einer Herzwand ein Verklemmen oder Herausrutschen des Rotors 2 aus dem Blutpumpengehäuse heraus verhindert.

Zusätzlich ist in Fig. 3 ein hydrodynamisches Lager 7 am auslassseitigen Ende der Blutpumpe gezeigt. Hierbei handelt es sich z.B. um ein Spiralrillenlager, das beispielsweise bei geringen Drehzahlen (bei denen die hydrodynamischen Eigenschaften noch nicht voll greifen) angrenzende Bereiche des Gehäuses der Blutpumpe berührt. Auch in diesem Bereich sind keramische und/oder DLC-Materialien zur Verringerung der Reibung bzw. Erhöhung der Lebensdauer vorgesehen. Bei Betriebsdrehzahl der Blutpumpe kommt es zur Wirkung des hydrodynamischen Lagers (d.h. keine direkte Berührung mehr). Der zusätzliche Axialschub des Rotors unterstützt und entlastet das hydrodynamische Lager. Auch für diesen Rotor gelten die im Patentanspruch 1 in eingereichter Form geltenden Merkmale.

Das vorliegende Schutzrecht betrifft außerdem die folgenden, teilweise oben bereits kurz angesprochenen, Aspekte:
Ein Aspekt ist, eine möglichst passiv gelagerte, beispielsweise axiale (bzw. halbaxiale oder radiale) Rotationsblutpumpe (kleine Steuereinheit) ohne großflächiges hydrodynamisches Lager zu entwickeln.

Diese Aufgabe wird beispielsweise gelöst, indem eine Axial/Halbaxial- bzw. Radialpumpe radial passiv magnetisch gelagert wird (eventuell mit einer unterstützenden hydrodynamischen Komponente) und auch axial eine passiv magnetische Vorspannung in eine Richtung gegeben ist. In die gegengesetzte axiale Richtung wirkt der sowieso vorhandene axiale Schub, der von der Druckdifferenz abhängig ist. Bei einer idealen Ausführung der Pumpe und entsprechender Adaptierung des Auslassgehäuses/Diffusors wird in einem menschlichen Kreislauf immer ein positiver axialer Schub vorhanden sein; somit kann der Rotor ohne zusätzliches Lager schweben. Unterstützt wird die axiale Lagerung durch ein einseitiges mechanisches oder hydrodynamisches Lager.

Ein einlassseitiges mechanisches Fanglager, wenn der Axialschub zu groß wird (während Ansaugens am Herzgewebe), kann durch Materialkombinationen (Keramik, Hartstoffbeschichtung, z.B. DLC) sichergestellt werden.

Die magnetische axiale Vorspannung kann auch so stark gewählt werden, dass auch in den Worst-Case-Szenarien (Ansaugen) ein Kontakt am axialen mechanischen Lager vorhanden ist.

Die radiale passive Magnetkraft und die Vorspannung in eine axiale Richtung können z.B. durch die in Fig. 1 gezeigte Magnetkonfiguration erreicht werden.

Die axiale Vorspannung kann auch durch zusätzliche anziehende bzw. abstoßende Magnete ausgeführt werden.

Die folgenden Weiterbildungen der vorliegenden Erfindung sind inter alia möglich:
1. Radiale Lagerung: Magnetisch unterstützte Journallager mit großem Spalt (z.B. ≥ 100 µm).
   Axiale Lagerung: passiv magnetisch (Vorspannung Richtung Auslass) und mechanisches Lager auslassseitig. Das mechanische Lager kann Axial-, Radial- oder andere Formen von Gleitlagern annehmen (siehe z.B. Fig. 2). Der Axialschub generiert Kräfte Richtung Einlass. Wenn notwendig: mechanisches (Fang-)Lager in Richtung Einlass.
2. Radiale Lagerung: Magnetisch unterstützte Gleitlager mit großem Spalt (>100 um).
   Axiale Lagerung: passiv magnetisch, Axialschub und - wenn notwendighydrodynamisches Lager. Das hydrodynamische Lager (siehe Fig. 3) kann als Spiralrillenlager oder durch Rampen etc. am Rotor oder am Gehäuse ausgeführt werden.
3. Blutpumpe, die im auslassseitigen Bereich des Rotors ein mechanisches Lager aufweist. Diese Pumpe hat die Magnete derart positioniert, dass die Pumpe ohne Vorleitrad auskommt. Um möglichst hohe Steifigkeiten zu erreichen, sind die rotorseitigen Ringmagnete möglichst nahe am Pumpenrohr mit den interagierenden Magneten angebracht. Die Ringmagnete sind durch Streben mit dem Rotor verbunden bzw. an den Schaufeln angebracht.
4. Blutpumpen, die ein mechanisches Lager aufweisen und magnetisch in Richtung Auslass vorgespannt sind. Auch in dieser Ausführung ist die Ringmagnetkonfiguration gleich der beschriebenen (Fig. 1). Auch hier wird durch den Axialschub Kraft vom mechanischen Lager weggenommen. Das mechanische Lager ist durch dünne, sich mit dem Rotor drehende Streben mit einer Spindel in der Mitte der Pumpe verbunden. Der Motor ist in den Ausführungen beispielsweise als Scheibenmotor ausgeführt, und die Motormagnete sind in der unteren Deckscheibe untergebracht. Die Pumpe kann auch mit einer oberen Deckscheibe ausgeführt werden, um zusätzliche, abstoßende mit dem Gehäuse interagierende Magnetkräfte auf den Rotor ausüben zu können. Eine derartige Pumpe muss immer einen mechanischen Kontakt des Kontaktlagers gewährleisten.

Im Folgenden werden ergänzend Fign. 4 bis 7 im Detail beschrieben:
Fig. 4 zeigt ein Beispiel einer axialen Blutpumpe 1 mit einem Rotor 2. Der Rotor weist eine Beschaufelung in Form von Schaufeln 14 auf. Der Rotor enthält nicht näher dargestellte Rotormagnete, die mit einem Motor bzw. Motorspulen 8 eines Stators der Blutpumpe 1 zusammenwirken. Die Rotormagnete können in den Schaufeln 14 untergebracht sein; alternativ können diese auch radial weiter innen im Rotor angebracht werden, wobei hierdurch der Wirkungsgrad des Motors gegebenenfalls etwas geringer ausfällt.

Der Stator ist im Wesentlichen rohrfömig mit einem Einlass 10 und einem nichtaxialen Auslass 11. Auf der dem Einlass abgewandten Seite des Rotors ist ein Lager 3 vorgesehen, das als Gleitlager, beispielsweise als Axial- oder Radiallager, ausgeführt ist. Das Lager ist so angeordnet, dass die durch den Einlass 10 einfließende Strömung das Lager auf Druck belastet. Die Schaufeln 14 sind so orientiert, dass bei einer fördernden Bewegung des Rotors 2 eine Druckentlastung auf das Lager 3 stattfindet. Zusätzlich zur radialen und axialen Stabilisierung des Rotors innerhalb des Stators der Blutpumpe sind Lagermagnete des Rotors 15 sowie Lagermagnete des Stators 16 vorgesehen. Die Lagermagnete des Rotors 15 sind mittels Streben 12 an Auslegern des Rotors befestigt. Die entsprechenden Ausleger, in denen die Lagermagnete 15 untergebracht sind, können z.B. kreisringförmig sein; es können auch einzelne Satelliten vorgesehen sein, die über den Kreisumfang verteilt angeordnet sind. Es können einfassseitig und auslassseitig jeweils solche Ausleger an entsprechenden Streben vorgesehen sein. Vorteilhaft an dieser Anordnung ist, dass (unabhängig von der Beschaufelung) eine gute radiale und axiale Stabilisierung des Rotors innerhalb des Stators erreicht werden kann. Hierzu ist es günstig, wenn ein Spalt 13 möglichst gering ist, beispielsweise ≥ 50 µm, aber geringer als 200 µm, vorzugsweise geringer als 150 µm, besonders vorzugsweise geringer als 100 µm.

Bei der in Fig. 4 gezeigten Anordnung wird durch die Streben 12 (die beispielsweise stabförmig sind und einen Flüssigkeitsdurchlass ermöglichen) erreicht, dass einerseits im Bereich der Ausleger bzw. der Lagermagnete 15/16 eine gute Stabilisierung des Rotors 2 gegeben ist, andererseits ein relativ ungehinderter Durchfluss von Fluid (vorzugsweise Blut) trotz der geringen Spaltbreiten 13 möglich ist.

Fig. 5 (hierbei zeigt Fig. 5a eine Seitansicht, Fig. 5b einen Schnitt B-B aus Fig. 5a, Fig. 5c einen Schnitt C-C- aus Fig. 5a sowie Fig. 5d eine verkleinerte perspektivische Ansicht der Gesamtpumpe mit Einlass 10 und Auslass 11; der Einlass 10 ist zur Anbringung an eine Kanüle bzw. ein Blutgefäß ausgebildet, Gleiches gilt für den Auslass 11) zeigt eine vollimplantierbare Blutpumpe mit einer intrakorporalen Radialpumpe mit Magneten und Motor innerhalb eines Pumpengehäuses. In dem Gehäuse ist ein axialer Einlass sowie ein radialer Auslass vorgesehen. Der Rotor 2 ist auf einem mechanischen Lager 4 gelagert, das als Kontaktlager ausgeführt ist und besonders reibungsarm ausgeführt ist, wobei die Materialpaarung beispielsweise keramische Materialien und/oder Hartstoffbeschichtung, z.B. DLC, enthält. Der Teil des Rotors, der gelagert ist, ist über Streben 12 mit dem übrigen Rotor verbunden. Der Rotor ist bereichsweise hohl ausgeführt, d.h., er enthält schräg/radiale Förderschaufeln, die axial durch den Einlass 10 eintretendes Fluid nach radial außen fördern. Der Rotor 2 ist im Wesentlichen scheibenförmig ausgeführt, wobei im unteren Bereich die Lagerung angeordnet ist und im Bereich darüber die für die Fluidförderung zuständigen Kanäle bzw. Schaufeln und oberhalb (d.h. in Richtung des Einlasses) Rotormagnete 18 vorgesehen sind, die mit axial darüber (d.h. noch weiter in Richtung des Einlasses 10 befindlichen) Motorspulen 17 zum Antrieb des im Stator befindlichen Rotors 2 zusammenwirken. Dies hat den Vorteil, dass möglichst wenige bewegliche Elemente vorgesehen sind und lediglich eine elektromagnetische Betätigung des Rotors innerhalb des Gehäuses der Blutpumpe stattfindet. Dies ist einerseits wartungsarm und andererseits einfach im Aufbau.

Außerdem sind rotorseitige Lagermagnete 15 sowie statorseitige Lagermagnete 16 vorgesehen, die zur radialen und axialen Stabilisierung des Rotors innerhalb des Gehäuses der Blutpumpe vorgesehen sind. Auch hier ist das Lager so angeordnet, dass bei Fluiddruck und stillstehendem Rotor der Rotor gegen das Lager 4 gepresst wird; bei Fluidförderung durch den Rotor 2 kommt es zu einer Druckentlastung des Lagers 4. Es sei nochmals darauf hingewiesen, dass der schichtenweise Aufbau des Rotors einen klaren und einfachen Aufbau ermöglicht, wobei Rotormagnete 18 und rotorseitige Lagermagnete 15 in einer Deckscheibe des Rotors untergebracht sind und mit entsprechenden statorseitigen Elementen (Motorspulen 17 bzw. statorseitigen Lagermagneten 16) zusammenwirken.

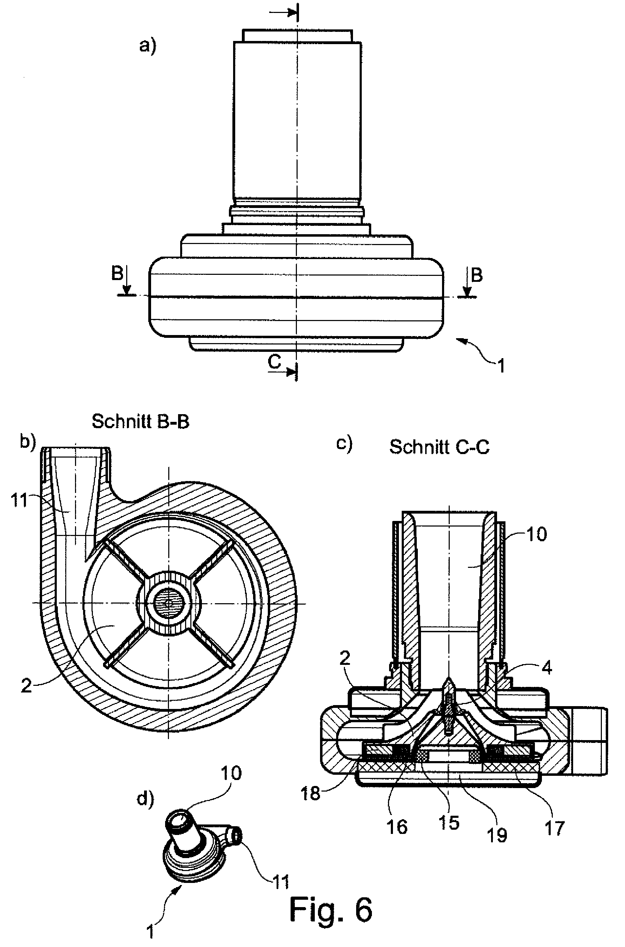

Fig. 6 (hierbei zeigt Fig. 6a eine Seitansicht, Fig. 6b einen Schnitt B-B aus Fig. 6a, Fig. 6c einen Schnitt C-C- aus Fig. 6a sowie Fig. 6d eine verkleinerte perspektivische Ansicht der Gesamtpumpe mit Einlass 10 und Auslass 11; der Einlass 10 ist zur Anbringung an eine Kanüle bzw. ein Blutgefäß ausgebildet, Gleiches gilt für den Auslass 11) zeigt eine weitere Ausführungsform, bei der wiederum ein axialer Einlass 10 und ein radialer Auslass 11 gezeigt sind. Es handelt sich hierbei um eine Halbaxialpumpe mit einem zentralen Statorelement, in dem statorseitige Lagermagnete 16 eingebaut sind und auf dessen Spitze die Kalotte eines mechanischen Lagers 4 sitzt. Die Motorspulen 17 befinden sich im Gehäuse, d.h. statorseitig in der Blutpumpe 1; diese wirken mit Rotormagneten 18, die im unteren Teil des Rotors 2 untergebracht sind, zusammen. Gegebenenfalls kann dieser Rotor 2 auch eine zusätzliche Deckscheibe enthalten. Es handelt sich also hierbei um eine Blutpumpe 1 mit einem Stator 19, wobei im Bereich einer Drehachse des Rotors 2 Lagermagnete des Stators angeordnet sind und wobei der Stator 19 im Bereich der Drehachse des Rotors 2 ein Statorelement aufweist, auf dessen Spitze eine Kalotte eines mechanischen Lagers angeordnet ist, und zusätzlich Rotormagnete 18 des Rotors vorzugsweise in einer Tragscheibe des Rotors 2 angeordnet sind und/oder mit den Rotormagneten in Wirkverbindung stehende Motorspulen 17 im Stator 19 auf der dem Einlass 10 abgewandten Seite des Rotors 2 angeordnet sind.

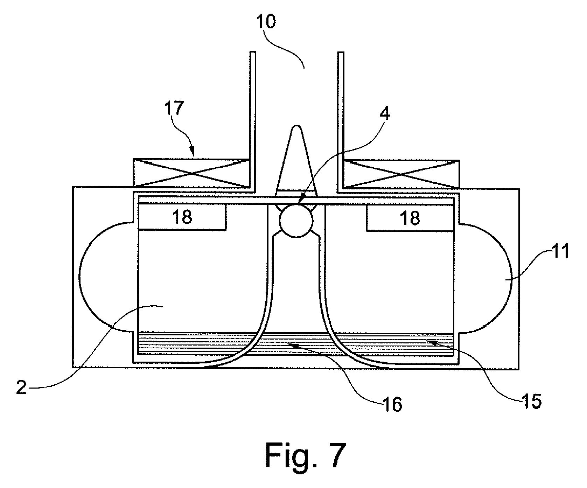

Fig. 7 zeigt eine weitere Ausführungsform einer Blutpumpe 1, bei der ein axialer Einlass 10 und ein radialer Auslass 11 gegeben sind. Der Rotor 2 weist auf der dem Einlass zugewandten Seite Rotormagnete 18 auf, die mit Motorspulen 17 im Gehäuse der Blutpumpe 1 zum Antrieb des Rotors zusammenwirken. Auf der dem Einlass abgewandten Seite des Rotors sind rotorseitige Lagermagnete 15 angebracht, die mit statorseitigen Lagermagneten 16 zur radialen und axialen Stabilisierung des Rotors innerhalb der Blutpumpe zusammenwirken. Zusätzlich ist ein mechanisches Lager 4 vorgesehen, bei dem Streben 12, die einen Flüssigkeitsdurchlass in Richtung vom Einlass 10 zum Auslass 11 erlauben, vorgesehen sind und eine zusätzliche mechanische Lagerung bzw. Abstützung des Rotors ermöglichen. Es handelt sich hierbei also um eine als Radialpumpe mit Streben 12 ausgeführte Blutpumpe 1, wobei auf einer Erhebung des Stators 19 im Bereich der Drehachse des Rotors 2 eine mit Streben 12 des Rotors 2 in Verbindung stehende Kalotte eines mechanischen Lagers angeordnet ist und Lagermagnete 16 einerseits statorseitig in dem Stator 19 angeordnet sind und wobei rotorseitige Lagermagnete 15 vorzugsweise auf der dem Einlass 10 abgewandten Seite des Rotors 2 und weiterhin vorzugsweise Rotormagnete 18 auf der dem Einlass zugewandten Seite des Rotors 2 angeordnet sind, die mit Motorspulen 17 im Bereich des Einlasses 10 der Blutpumpe zusammenwirken.

Das vorliegende Schutzrecht betrifft unter anderem die folgenden Aspekte:
1. Blutpumpe (1), enthaltend
   einen Einlass (10),
   einen Auslass (11) sowie
   einen Rotor (2) zur Förderung von Fluid vom Einlass (10) zum Auslass (11), wobei der Rotor (2) innerhalb der Blutpumpe (1) radial passiv magnetisch gelagert ist und axial in Richtung des Auslasses (11) zumindest passiv magnetisch angezogen ist, vorzugsweise derart, dass bei fluidfördernder Drehung des Rotors (2) der Axialschub des Rotors entgegen der axial in Richtung des Auslasses wirkenden magnetischen Anziehung wirkt.
2. Blutpumpe nach Aspekt 1, dadurch gekennzeichnet, dass der Rotor (2) rein passiv axial magnetisch angezogen ist.
3. Blutpumpe nach einem der vorhergehenden Aspekte, dadurch gekennzeichnet, dass zur Verstärkung des Axialschubs eine auf den Rotor (2) wirkende elektromagnetische und/oder elektrodynamische Vorrichtung vorgesehen ist.
4. Blutpumpe nach einem der vorhergehenden Aspekte, dadurch gekennzeichnet, dass der Rotor (2) insbesondere zur axialen Lagerung zusätzlich durch ein mechanisches Lager (4) unterstützt ist.
5. Blutpumpe nach Aspekt 4, dadurch gekennzeichnet, dass das mechanische Lager (4) im einlassseitigen und/oder auslassseitigen Bereich des Rotors (2) angeordnet ist.
6. Blutpumpe nach einem der Aspekte 4 oder 5, dadurch gekennzeichnet, dass das mechanische Lager (4) als Kontaktlager, insbesondere als Spitzen- oder Balllager, ausgeführt ist.
7. Blutpumpe nach einem der vorhergehenden Aspekte, dadurch gekennzeichnet, dass der Rotor (2) insbesondere zur axialen Lagerung zusätzlich durch ein hydrodynamisches Lager (7) unterstützt ist.
8. Blutpumpe nach Aspekt 7, dadurch gekennzeichnet, dass das hydrodynamische Lager (7) im auslassseitigen Bereich des Rotors (2) angeordnet ist.
9. Blutpumpe nach einem der Aspekte 7 oder 8, dadurch gekennzeichnet, dass das hydrodynamische Lager (7) als Spiralrillenlager ausgeführt ist.
10. Blutpumpe nach einem der vorhergehenden Aspekte, dadurch gekennzeichnet, dass ein zusätzliches Lager, insbesondere ein axiales Fanglager zur Begrenzung der axialen Rotorbewegung in Richtung des Einlasses (10), vorgesehen ist.
11. Blutpumpe nach einem der Aspekte 4 bis 10, dadurch gekennzeichnet, dass im Bereich mindestens eines zusätzlichen Lagers der Rotor (2) und/oder angrenzende Teile der Blutpumpe, insbesondere mit keramischen Materialien und/oder DLC, verstärkt sind.

Die im vorliegenden Schutzrecht gezeigte Lagertechnologie kann eine Plattform für kleine, passive Rotationsblutpumpen mit hoher Hämokompatibilität und kleiner Steuereinheit etc. darstellen.

### Bezugszeichenliste

(Auszug, wenn nicht anders bezeichnet, für alle Ausführungsformen in den Zeichnungen gültig)
- 1: Blutpumpe
- 2: Rotor
- 3: Lager
- 4: Kontaktlager
- 5: Streben (vom Pumpenrohr ausgehend)
- 6: Richtung eines Pfeils
- 7: hydrodynamisches Lager / Spiralrillenlager
- 8: Motor
- 9: Fanglager
- 10: Einlass
- 11: Auslass
- 12: Streben (am Rotor)
- 13: Spalt
- 14: Schaufeln des Rotors
- 15: Lagermagnete Rotor
- 16: Lagermagnete Stator
- 17: Motorspule
- 18: Rotormagnete
- 19: Stator

## Patentansprüche

1. Blutpumpe (1), enthaltend
einen Einlass (10),
einen Auslass (11) sowie
einen Rotor (2) zur Förderung von Fluid vom Einlass (10) zum Auslass (11), wobei der Rotor (2) innerhalb der Blutpumpe (1) radial passiv magnetisch gelagert ist und axial in eine Richtung zumindest passiv magnetisch angezogen oder abgestoßen ist, vorzugsweise derart, dass bei fluidfördernder Drehung des Rotors (2) der Axialschub des Rotors entgegen der axial in Richtung des Auslasses wirkenden magnetischen Anziehung wirkt.

2. Blutpumpe nach Anspruch 1, **dadurch gekennzeichnet, dass** der Rotor (2) rein passiv axial magnetisch angezogen ist.

3. Blutpumpe nach einem der vorhergehenden Ansprüche, **dadurch gekennzeichnet, dass** zur Verstärkung des Axialschubs eine auf den Rotor (2) wirkende elektromagnetische und/oder elektrodynamische Vorrichtung vorgesehen ist.

4. Blutpumpe nach einem der vorhergehenden Ansprüche, **dadurch gekennzeichnet, dass** der Rotor (2) insbesondere zur axialen Lagerung zusätzlich durch ein mechanisches Lager (4) unterstützt ist,
wobei vorzugsweise das mechanische Lager (4) im einlassseitigen und/oder auslassseitigen Bereich des Rotors (2) angeordnet ist und/oder
wobei vorzugweise das mechanische Lager (4) als Gleitlager, insbesondere als Axial- und/oder Radiallager, ausgeführt ist.

5. Blutpumpe nach einem der vorhergehenden Ansprüche, **dadurch gekennzeichnet, dass** der Rotor (2) insbesondere zur axialen Lagerung zusätzlich durch ein hydrodynamisches Lager (7) unterstützt ist,
wobei vorzugsweise das hydrodynamische Lager (7) im auslassseitigen Bereich des Rotors (2) angeordnet ist und/oder
wobei vorzugsweise das hydrodynamische Lager (7) als Spiralrillenlager ausgeführt ist.

6. Blutpumpe nach einem der vorhergehenden Ansprüche, **dadurch gekennzeichnet, dass** ein zusätzliches Lager, insbesondere ein axiales Fanglager zur Begrenzung der axialen Rotorbewegung in Richtung des Einlasses (10), vorgesehen ist.

7. Blutpumpe nach einem der Ansprüche 4 bis 6, **dadurch gekennzeichnet, dass** im Bereich mindestens eines zusätzlichen Lagers der Rotor (2) und/oder angrenzende Teile der Blutpumpe, insbesondere mit keramischen Materialien und/oder Hartstoffbeschichtung, beispielsweise DLC, verstärkt sind.

8. Blutpumpe nach einem der vorhergehenden Ansprüche, **dadurch gekennzeichnet, dass** die Blutpumpe eine voll in einen menschlichen oder tierischen Körper implantierbare Pumpe ist und/oder
dass der Einlass der Pumpe axial bezüglich einer Drehachse des Rotors (2) ist und der Auslass radial bezüglich der Drehachse des Rotors (2) ist und/oder
der Rotor (2) der Blutpumpe axial, halbaxial oder radial ausgelegt ist.

9. Blutpumpe nach einem der vorhergehenden Ansprüche, **dadurch gekennzeichnet, dass** Lagermagnete (15) des Rotors (2) zur radialen und axialen Stabilisierung des Rotors innerhalb des Gehäuses der Blutpumpe (1) vorgesehen sind.

10. Blutpumpe nach Anspruch 9, **dadurch gekennzeichnet, dass** die Lagermagnete (15) auf der dem Einlass (10) abgewandten Seite des Rotors (2) angeordnet sind oder
dass die Lagermagnete (15) auf der dem Einlass (10) zugewandten Seite des Rotors (2) angeordnet sind.

11. Blutpumpe nach einem der vorhergehenden Ansprüche, **dadurch gekennzeichnet, dass** Motorspulen (17) im Stator (19) der Blutpumpe und Rotormagnete (18) im Rotor (2) vorgesehen sind, wobei die Motorspulen (17) auf der dem Einlass (10) zugewandten Seite des Rotors (2) oder
auf der dem Einlass (10) abgewandten Seite des Rotors (2) angeordnet sind.

12. Blutpumpe nach einem der vorhergehenden Ansprüche, insbesondere nach einem der Ansprüche 4 bis 11, **dadurch gekennzeichnet, dass** ein mechanisches Lager (4) auf der dem Einlass (10) zugewandten Seite des Rotors (2) und/oder
auf der dem Einlass (10) abgewandten Seite des Rotors (2) angeordnet ist.

13. Blutpumpe nach einem der vorhergehenden Ansprüche, **dadurch gekennzeichnet, dass** der Rotor (2) in seinem zentralen Bereich über Streben (12) mit einem mechanischen Lager (4) verbunden ist.

14. Blutpumpe nach einem der vorhergehenden Ansprüche, **dadurch gekennzeichnet, dass** die Blutpumpe (1) mit einem Stator (19) ausgeführt ist, wobei im Bereich einer Drehachse des Rotors (2) Lagermagnete (16) des Stators angeordnet sind und wobei der Stator im Bereich der Drehachse des Rotors ein Statorelement aufweist, auf dessen Spitze eine Kalotte eines mechanischen Lagers angeordnet ist, und zusätzlich Rotormagnete (18) des Rotors vorzugsweise in einer Tragscheibe des Rotors (2) angeordnet sind, und/oder
mit den Rotormagneten in Wirkverbindung stehende Motorspulen (17) im Stator auf der dem Einlass (10) abgewandten Seite des Rotors (2) angeordnet sind.

15. Blutpumpe nach einem der Ansprüche 1 bis 13, **dadurch gekennzeichnet, dass** die Blutpumpe (1) als Radialpumpe mit Streben ausgeführt ist, wobei auf einer Erhebung des Stators (19) im Bereich der Drehachse des Rotors (2) eine mit Streben (12) des Rotors (2) in Verbindung stehende Kalotte eines mechanischen Lagers (4) angeordnet ist und Lagermagnete (16) einerseits statorseitig in dem Stator angeordnet sind und rotorseitige Lagermagnete (15), vorzugsweise auf der dem Einlass (10) abgewandten Seite des Rotors (2), angeordnet sind und weiterhin vorzugsweise Rotormagnete (18) auf der dem Einlass (10) zugewandten Seite des Rotors (2) angeordnet sind, die mit Motorspulen (17) im Bereich des Einlasses (10) der Blutpumpe (1) zusammenwirken.
